# EUROPÄISCHE PATENTANMELDUNG

(11) **EP 1 974 699 A1**
(43) Veröffentlichungstag der Anmeldung: **01.10.2008**
(21) Anmeldenummer: 08005805.0
(22) Anmeldetag: 27.03.2008
(51) Int. Cl.: A61F 2/68

(54) **Prothesen- oder Orthesengelenk**

(30) Priorität: 29.03.2007 DE 102007015560
(71) Anmelder: Otto Bock HealthCare IP GmbH & Co. KG, 37115 Duderstadt (DE)
(72) Erfinder: Boiten, Herman, 37085 Göttingen (DE); Nörthemann, Jens, 37115 Duderstadt (DE)
(74) Vertreter: Gramm, Werner

(57) **Zusammenfassung**

Prothesen- oder Orthesengelenk mit zumindest zwei Gelenkteilen (2), die zueinander um eine Gelenkachse (3) schwenkbar gelagert sind, und einem Schwenkkolben (6), der drehfest an einem der Gelenkteile (2) angeordnet und in einer ein Fluid enthaltenden Verdrängerkammer (5) geführt ist, wobei der Schwenkkolben (6) die Verdrängerkammer (5) in zwei Teilkammern unterteilt, die über einen Verbindungskanal (9) zumindest über einen gewählten Verschwenkwinkelbereich des Schwenkkolbens miteinander verbunden sind, wobei zwischen dem Schwenkkolben (6) und einer Verdrängerkammerwandung zumindest eine strömungsquerschnittbestimmende Kontur (64) ausgebildet ist, der über einen gewählten Verschwenkwinkel in strömungstechnischer Verbindung mit dem Verbindungskanal (9) und der Verdrängerkammer (5) steht und in Abhängigkeit von der Winkelstellung des Schwenkkolbens (6) einen unterschiedlichen freien Strömungsquerschnitt für den Durchtritt des Fluides bereitstellt.

## Beschreibung

Die Erfindung betrifft ein Prothesen- oder Orthesengelenk mit einem ersten und einem zweiten Gelenkteil, die zueinander um eine Gelenkachse schwenkbar gelagert sind und einem Schwenkkolben, der drehfest an dem Gelenkober- oder Gelenkunterteil angeordnet ist und in einer ein Fluid enthaltenen Verdrängerkammer geführt ist, wobei der Schwenkkolben die Verdrängerkammer in zwei Teilkammern unterteilt, die über einen Verbindungskanal zumindest über einen gewählten Verschwenkwinkelbereich des Schwenkkolbens miteinander verbunden sind.

Die Erfindung ist insbesondere für die Steuerung der Bewegung eines Kniegelenkes oder eines Fußgelenkes geeignet. Grundsätzlich sind jedoch auch andere Anwendungsgebiete in der Orthetik oder Prothetik denkbar, beispielsweise bei Hüftgelenksprothesen oder -orthesen.

Aus dem Stand der Technik sind so genannte Rotationshydrauliken bekannt, bei denen Gelenkteile einer Prothese oder Orthese verschwenkbar miteinander verbunden sind und ein Schwenkkolben eine Verdrängerkammer in zwei Teilkammern unterteilt. Die Teilkammern sind über parallel geschaltete, entgegengesetzt wirkende Drosselrückschlagventile miteinander verbunden. Eine Veränderung des Strömungsdurchmessers des Verbindungskanals kann dabei von außen über eine axial verschiebbare Drosselstange erfolgen, so dass das Fluid, insbesondere Hydrauliköl, nur mit einem höheren Strömungswiderstand von der einen Teilkammer in die andere Teilkammer während einer Flexions- oder Extensionsbewegung strömen kann. Eine solche Gelenkeinrichtung ist in der DE 43 38 946 C1 beschrieben. Eine Querschnittsveränderung des Verbindungskanals kann dabei in Abhängigkeit von einer Axialkraft, die beispielsweise beim Auftreten entsteht, bewirkt werden. Dies erfordert eine axial bewegliche Lagerung eines Drosselstabes.

Herkömmliche Rotationshydrauliken weisen eine über den Weg konstante Dämpfung auf. Bei einem höhere Energieeintrag in den Dämpfer, beispielsweise als Folge schnelleren Gehens, folgt daraus eine erhöhte Flexion oder Extension. Der erwünschte Beugewinkel oder Streckwinkel wird somit überschritten.

Aufgabe der vorliegenden Erfindung ist es, ein Prothesen- oder Orthesengelenk bereitzustellen, mit dem auf einfache Art und Weise eine angepasste Dämpfung der Verschwenkbewegung erzielt werden kann.

Erfindungsgemäß wird diese Aufgabe durch die Merkmale des Anspruchs 1 gelöst. Vorteilhafte Ausgestaltungen und Weiterbildungen der Erfindung sind in den Unteransprüchen beschrieben.

Das erfindungsgemäße Prothesen- oder Orthesengelenk mit zumindest zwei Gelenkteilen, die zueinander um eine Gelenkachse schwenkbar gelagert sind, und einem Schwenkkolben, der drehfest an dem Gelenkober- oder Gelenkunterteil angeordnet und in einer ein Fluid enthaltenen Verdrängerkammer geführt ist, wobei der Schwenkkolben die Verdrängerkammer in zwei Teilkammern unterteilt, die über eine Verbindungskammer zumindest über einen gewählten Verschwenkwinkelbereich des Schwenkkolbens miteinander verbunden sind, sieht vor, dass zwischen dem Schwenkkolben und der Verdrängerkammer zumindest eine strömungsquerschnittsbestimmende Kontur ausgebildet ist, die über einen gewählten, eingestellten oder festgelegten Verschwenkwinkel in strömungstechnischer Verbindung mit dem Verbindungskanal und der Verdrängerkammer steht und in Abhängigkeit von der Winkelstellung des Schwenkkolbens einen unterschiedlichen freien Strömungsquerschnitt für den Durchtritt des Fluides bereitstellt. Durch das erfindungsgemäße Prothesengelenk bzw. Orthesengelenk ist es möglich, den Fluid- oder Ölfluss während der Bewegung der Gelenkteile zueinander, zum Beispiel des Oberteils relativ zu dem Unterteil des Gelenkes, in Abhängigkeit von der Winkelstellung, also während der Bewegung, zu verändern, so dass ein Fluidstrom von der einen Teilkammer in die andere Teilkammer bereitgestellt wird, der je nach Stellung des Gelenkes und damit des Schwenkkolbens modifiziert ist. Durch die Veränderung des freien Strömungsquerschnittes für das Fluid durch die Kontur zwischen dem Schwenkkolben und der Verdrängerkammerwandung, zum Beispiel durch einen Kanal in dem Schwenkkolben und/oder in der Verdrängerkammer, ist es möglich, eine winkelabhängige Dämpfung auf einfache Weise bereitzustellen, so dass eine Rotationshydraulik in dem Gelenk realisiert wird, die eine höhere Dämpfung bei einer bestimmten Winkelstellung bereitstellt.

Eine Weiterbildung der Erfindung sieht vor, dass zwei Konturen zwischen der Verdrängerkammer und dem Schwenkkolben ausgebildet bzw. eingearbeitet sind, die den jeweiligen Einmündungen des Verbindungskanals zugeordnet sind, so dass ein Fluidstrom sowohl in Extensionsrichtung als auch in Flexionsrichtung über die Einmündungen des Verbindungskanals und die Kontur innerhalb des Schwenkkolbens oder der Verdrängerkammer in die Teilkammer einströmen kann. Dadurch ist es möglich, sowohl in Extensionsrichtung als auch in Flexionsrichtung beugewinkelabhängige, veränderliche Dämpfungen bereitzustellen, da separat der freie Strömungsquerschnitt für den Durchtritt des Fluides verändert wird. Zur Bereitstellung unterschiedlicher und über den Winkelverlauf der Verschwenkung variabler Dämpfungen hinsichtlich Extension und Flexion sind unterschiedliche Konturen für Extension und Flexion vorgesehen, so dass durch ein Einarbeiten der Konturen oder Kanäle in den Schwenkkolben oder in das die Verdrängerkammer bildende Gehäuse eine schwenkwinkelabhängige und schwenkrichtungsabhängige Veränderung der Dämpfung bereitgestellt werden kann.

In einer Ausgestaltung der Erfindung ist es vorgesehen, dass die Kontur oder der Kanal in einer Achsfläche des Schwenkkolbens eingearbeitet ist. Bei mehreren Konturen oder Kanälen ist es vorgesehen, dass diese voneinander getrennt in der Achsfläche des Schwenkkolbens eingearbeitet sind, wodurch auf einfache Art und Weise, beispielsweise durch Einfräsen oder Einschleifen der Kontur oder Konturen beziehungsweise des Kanals oder der Kanäle in die Achsfläche des Schwenkkolbens, eine Grundform einer Dämpfungskurve festgelegt werden kann. Die Achsfläche des Schwenkkolbens ist dabei diejenige Fläche, die nicht als Kolbenplatte ausgestaltet ist und im Wesentlichen als Kreiszylinder ausgebildet ist. Die Achsfläche gleitet auf einer korrespondierend ausgebildeten Kontur der Verdrängerkammer und dichtet die Teilkammern auf der der Kolbenplatte gegenüberliegende Seite des Schwenkkolbens gegeneinander ab. Alternativ oder ergänzend zu einer Kontur in der Achsfläche kann diese auch in der Verdrängerkammer, genauer in der der Achsfläche zugeordneten Wandung des die Verdrängerkammer ausbildenden Gehäuses ausgebildet sein, so dass durch die Kontur in der Verdrängerkammer beispielsweise eine Grunddämpfung bereitgestellt wird, die durch den Einbau eines angepassten Schwenkkolbens modifiziert und an die Bedürfnisse des Prothesen- oder Orthesennutzers angepasst wird.

In einer Variante der Erfindung kann die Kontur die Einmündung des Verbindungskanals in Abhängigkeit von der Winkelstellung des Schwenkkolbens in einem unterschiedlichen Maße überdecken, also den Zustrom in die Kontur durch ein teilweises oder vollständiges Verschließen der Einmündung verringern oder unterbinden. Die Kontur überdeckt dann nicht während der gesamten Verschwenkbewegung, in der eine strömungstechnische Verbindung zwischen dem Verbindungskanal und der Kontur besteht, die Einmündung, vielmehr wird nur ein begrenzter Querschnitt der Einmündung zur Überdeckung mit dem durch die Kontur ausgebildeten Strömungskanal gebracht, so dass eine Veränderung des freien Strömungsquerschnittes an der Schnittstelle zwischen der Kontur und der Einmündung bewirkt wird. Bei einer radial ausgerichteten Einmündung und einer in Umfangsrichtung angeordneten oder ausgebildeten Kontur wird die sich verändernde Überdeckung durch eine axiale Verlagerung der Kontur erreicht. Bei einer axialen Anordnung der Einmündung wird die Veränderung durch eine entsprechend spiralförmige Anordnung und Ausgestaltung der Kontur oder des Kanals zur partiellen Überdeckung und Veränderung des wirksamen Strömungsquerschnittes bewirkt.

Eine Weiterbildung der Erfindung sieht vor, dass die Kontur oder der Kanal die Einmündung zumindest für einen festgelegten Schwenkwinkelbereich vollständig überdeckend ausgebildet ist und dass der Kontur- oder Kanalgrund in Abhängigkeit von der Winkelstellung des Schwenkkolbens einen unterschiedlichen Abstand zu der Einmündung aufweist. Je größer der Abstand zwischen dem Kontur- oder Kanalgrund und der Einmündung ausgebildet ist, desto größer ist der freie Strömungsquerschnitt. Wird der Konturgrund sehr nahe an die Einmündung herangeführt, verengt sich im Übergangsbereich der Strömungsquerschnitt, so dass nur eine geringe Menge an Fluid durch diese Engstelle hindurchtreten kann, was zu einer erhöhten Dämpfung führt. Der Vorteil einer solchen Anordnung liegt bei einer radial ausgerichteten Einmündung darin, dass der Kanal oder die Kontur geradlinig ausgebildet sein kann, lediglich die Tiefe der Ausfräsung der Kontur oder des Kanals wird verändert, um unterschiedliche Dämpfungsraten bereitzustellen. Eine solche Ausgestaltung kann auch mit einer Kanalkontur oder einem Kanalverlauf kombiniert werden, die den Mündungsquerschnitt und das Maß der Überdeckung zu der Einmündung verändert.

Grundsätzlich ist es möglich, dass mehrere Einmündungen des Verbindungskanals in die Teilkammern vorhanden sind, insbesondere können zwei Einmündungen in jeder Teilkammer vorhanden sein, durch die das Fluid von der einen Teilkammer durch den Verbindungskanal in die zweite Teilkammer hindurchströmen kann. Die weiteren Einmündungen können entweder in Serie oder parallel zu den mit der Kontur bzw. den Konturen oder dem Kanal bzw. den Kanälen in Verbindung stehenden Einmündungen angeordnet sein, um ein abgestimmtes Dämpfungsverhalten bereitstellen zu können.

Zur Einstellung der Dämpfung ist es vorgesehen, dass in dem Verbindungskanal zumindest ein Rückschlagventil angeordnet ist, bevorzugt sind mehrere Rückschlagventile für die Flexion und die Extension angeordnet, die sich gegenseitig nicht beeinflussen, so dass für beide Bewegungsrichtungen eine entsprechende Steuerung der Dämpfung bereitgestellt werden kann.

Weiterhin besteht die Möglichkeit, dass zumindest ein Schaltventil in dem Verbindungskanal eingebaut ist, um hohe Dämpfungswerte, insbesondere in Flexions- oder Beugerichtung zu erzeugen, die unabhängig von der Kontur und der Dämpfungswirkung der Kanäle in dem Schwenkkolben über den gesamten Bewegungsbereich wirksam sind. Durch das Schaltventil kann auch die Dämpfung nahezu vollständig aufgehoben werden, um beispielsweise für das Fahrradfahren eine von der Winkelstellung unabhängige, minimale Dämpfung zu erreichen.

Um ein Grundniveau der Dämpfung einstellen zu können, ist in dem Verbindungskanal zumindest eine einstellbare Drossel angeordnet, die bevorzugt von außen verstellbar ausgebildet ist, um so eine Anpassung des Prothesen- oder Orthesengelenkes an den jeweiligen Gelenknutzer vornehmen zu können.

Bei einer Anordnung der Konturen oder Kanäle in der Achsfläche des Schwenkkolbens wird der Weg bzw. die Winkelverstellbarkeit durch die Länge der Dichtfläche begrenzt. Bei Rotationshydrauliken ist dies diejenige Fläche, die direkt auf der Schwenkachse liegt. Ist der gewünschte Schwenkwinkel, über den die Dämpfung variiert werden soll, ohne dass eine Verbindung zwischen den beiden Teilkammern entsteht, größer als die Dichtfläche, kann der gewünschte Weg bzw. der Verschwenkwinkel durch eine Parallelschaltung mehrerer Kanäle erreicht werden. Bei einer Parallelschaltung ist dann der erste Kanal kürzer als die Länge der Dichtfläche zwischen der Achsfläche und der Einmündung.

Nachfolgend werden Ausführungsbeispiele der Erfindung anhand der beigefügten Figuren näher erläutert. Es zeigen:
- Figur 1 -: ein Prothesengelenk in Seitenansicht;
- Figur 2 -: eine Schnittansicht eines Prothesengelenkes
- Figur 3 -: eine Teilansicht eines Prothesengelenkes in schematischer Darstellung;
- Figur 4 -: eine Einzeldarstellung eines Schwenkkolbens;
- Figur 4a -: eine Variante der Figur 4;
- Figur 5 -: eine Seitenansicht eines eingebauten Schwenkkolbens mit einem Schaltbild;
- Figur 6 -: einen Strömungsverlauf bei beginnender Flexion;
- Figur 7 -: einen Strömungsverlauf bei fortgeschrittener Flexion;
- Figur 8 -: einen Strömungsverlauf bei einer Stellung gemäß Figur 7 in Extensionsrichtung;
- Figur 9 -: eine Stellung am Ende der Extension;
- Figur 10 -: eine Schaltstellung in der Standphase; sowie
- Figur 11 -: ein exemplarischer Dämpfungsverlauf.

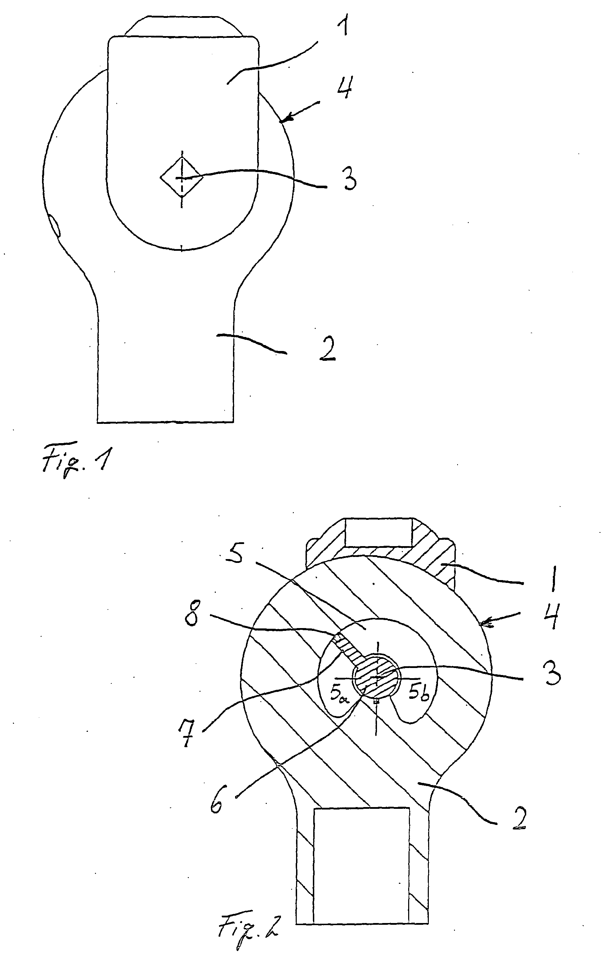

In der Figur 1 ist ein Prothesengelenk, vorliegend ein Prothesenkniegelenk, in Seitenansicht dargestellt. Das Prothesenkniegelenk weist zwei Gelenkteile 1, 2, nämlich ein Gelenkoberteil 1 und Gelenkunterteil 2 auf, die schwenkbar um eine Achse 3 miteinander verbunden sind. Grundsätzlich sind auch Ausführungsformen mit mehr als zwei Gelenkteilen 1, 2 möglich und vorgesehen. Nachfolgend wird aus Gründen der Anschaulichkeit von einem Gelenkober- und -unterteil 1, 2 gesprochen. Sowohl an dem Gelenkoberteil 1 als auch an dem Gelenkunterteil 2 sind nicht näher dargestellte Anschlussmittel vorgesehen, mit denen das Prothesenkniegelenk einerseits an dem Prothesennutzer befestigt und andererseits mit weiteren Protheseneinrichtungen versehen werden kann. In der dargestellten Ausführungsform stützt sich das Gelenkoberteil 1 auf einer zylindrischen Oberfläche 4 des Gelenkunterteils 2 ab. Aufgrund einer exzentrischen Anordnung der Achse 3 in dem Gelenkunterteil 2 läuft das Gelenkoberteil 1 auf der Oberfläche 4 des Gelenkunterteils 2 auf, so dass ein separater Gelenkanschlag entfallen kann.

In der Figur 2 ist das Prothesenkniegelenk in einer Schnittdarstellung gezeigt. In dem Gelenkunterteil 2 ist eine Verdrängerkammer 5 ausgebildet, in der ein Schwenkkolben 6 verschwenkbar gelagert ist. Der Schwenkkolben 6 ist dabei drehfest an dem Gelenkoberteil 1 gelagert und wird zusammen mit dem Gelenkoberteil 1 relativ zu dem Gelenkunterteil 2 verschwenkt, wenn das Gelenk bewegt wird. Grundsätzlich ist auch eine umgekehrte Anordnung möglich. Der Schwenkkolben 6 verschwenkt um die Drehachse 3 und weist einen im Wesentlichen zylindrischen Grundkörper und eine daran angeformte Kolbenplatte 7 auf, die sich radial von der Schwenkachse 3 weg bis zum Rand der Verdrängerkammer 5 erstreckt. An dem radial äußeren Ende der Kolbenplatte 7 ist eine Dichtleiste 8 angeordnet. Die Kolbenplatte 7 teilt die Verdrängerkammer 5 in zwei Teilkammern 5a, 5b, die Dichtleiste 8 dichtet die beiden Teilkammern 5a, 5b gegeneinander ab. An dem Grundkörper 6 kann ebenfalls ein Dichtelement anliegen, um eine möglichst vollständige Abdichtung der beiden Teilkammern 5a, 5b zueinander zu erreichen, so dass kein Leckstrom zwischen den Teilkammern 5a, 5b entsteht. Um das Gelenkoberteil 1 überhaupt gegenüber dem Gelenkunterteil 2 verschwenken zu können, ist ein Verbindungskanal 9 zwischen den Teilkammern 5a und 5b vorgesehen, der später erläutert wird. Ohne Verbindungskanal 9 würde eine Verschwenkung des Schwenkkolbens 6 nur über einen Fluidtransport mittels Leckstrom erfolgen, was praktisch eine Blockierung des Gelenkes bedeuten würde.

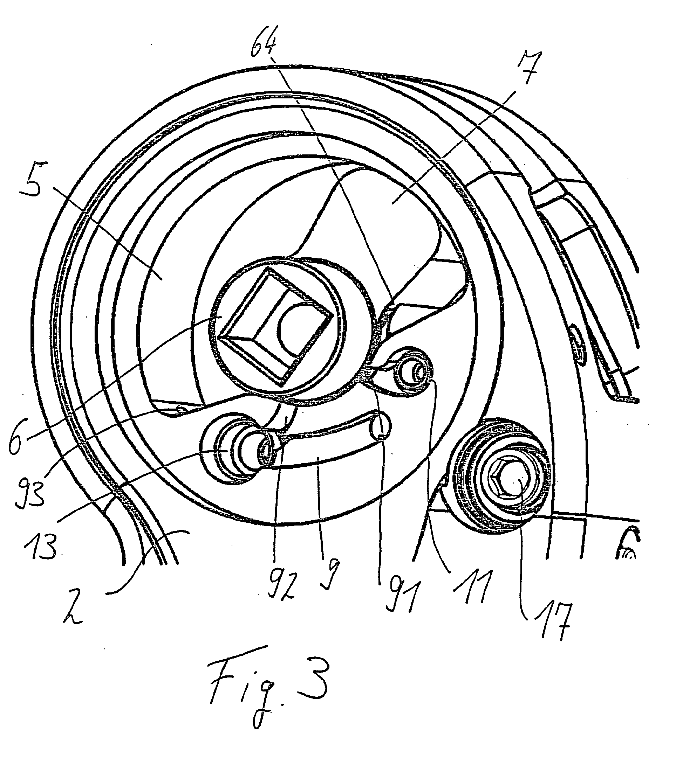

Die Figur 3 zeigt eine Teildarstellung des Prothesen- oder Orthesengelenkes ohne das Gelenkoberteil 1 in einer teilmontierten Darstellung. Das Gelenkunterteil 2 ist mit der Verdrängerkammer 5 und dem darin schwenkbar gelagerten Drehkolben 6 mit der Kolbenplatte 7 dargestellt. In dem Gelenkunterteil 2 sind Rückschlagventile 11, 13 angeordnet, die eine Verschwenkung des Gelenks in Streckrichtung verhindern, eine Flexion jedoch ermöglichen. In dem Gelenkunterteil 2 ist ein Verbindungskanal 9 eingearbeitet, durch den Fluid aus der Verdrängerkammer 5 hindruchströmen kann, so dass ein Stoffstrom des Fluides zwischen den Teilkammern 5a, 5b entlangströmen kann. Weiterhin ist ein Flexionsventil 17 zu erkennen, das später erläutert wird.

Analog zu einer möglichen Flexion ist es vorgesehen, dass entsprechende Ventile oder Drosseln für eine Extension vorhanden sind, die ebenfalls über den Verbindungskanal 9 eine strömungstechnische Verbindung der beiden Teilkammern 5a, 5b herstellen.

Innerhalb des Schwenkkolbens 6 sind an einer Achsfläche 63, die diejenige Fläche des Grundkörpers darstellt, die im Wesentlichen zylindrisch ausgebildet ist und in einer korrespondierenden Aufnahme des Gelenkunterteils 2 entlanggleitet, sind Konturen in Gestalt von Kanälen eingearbeitet, von denen ein Flexionskanal 64 in der Figur 3 zu erkennen ist. Der Verbindungskanal 9 verzweigt sich mehrfach und bildet mehrere Einmündungen 91, 92, 93 in der Gehäusewandung der Verdrängerkammer 5 aus, wobei nicht sämtliche Einmündungen in der Figur 3 zu erkennen sind.

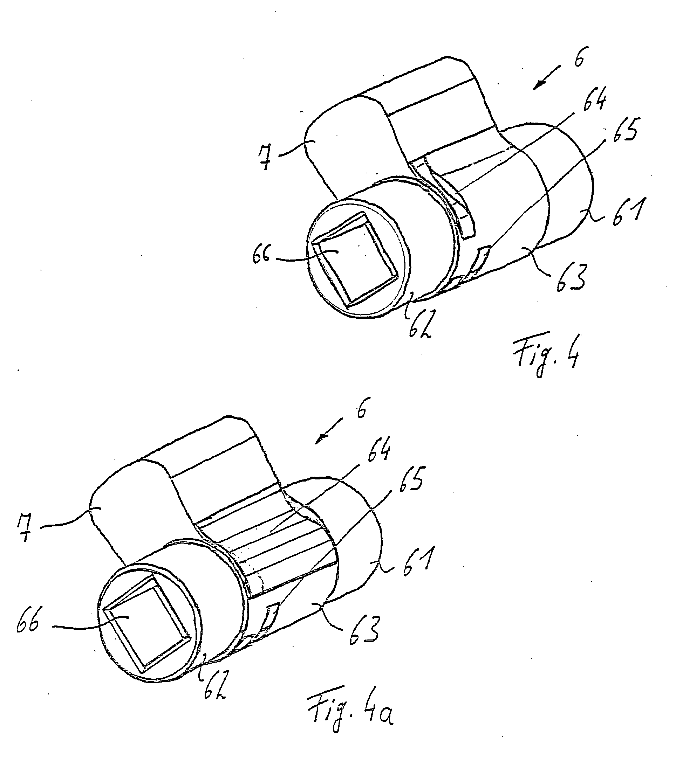

In der Figur 4 ist ein Schwenkkolben 6 in Einzeldarstellung gezeigt. Neben der Kolbenplatte 7 ist die Achsfläche 3 mit den eingearbeiteten Kanälen 64, 65 zu erkennen. Die Achsfläche 3 liegt zwischen zwei Zapfen 61, 62, auf denen der Schwenkkolben 6 innerhalb des Gelenkunterteils 2 gelagert ist. An den Stirnseiten des Schwenkkolbens 6 sind viereckige Ausnehmungen 66 ausgebildet, um das Gelenkoberteil 1 formschlüssig und drehfest an dem Schwenkkolben 6 festzulegen.

Die Kanäle 64, 65 sind an dem radialen Umfang der Achsfläche 63 eingearbeitet, bevorzugt eingefräst, wobei in der dargestellten Ausführungsform die Kanäle 64, 65 gerade ausgebildet sind, so dass sie während der gesamten Winkelstrecke, in der die Kanäle 64, 65 den jeweils zugeordneten Einmündungen 91, 92 gegenüberliegen und diese vollständig überdecken. Wie anhand des Flexionskanals 64 zu erkennen ist, weist der Kanal 64 eine sich verändernde Tiefe auf, wodurch der freie Strömungsquerschnitt in Abhängigkeit von der Winkelstellung des Schwenkkolbens 6 und damit des Gelenkoberteils 1 verändert wird. Im Bereich des von der Kolbenplatte 7 abgewandten Endes des Kanals 64 nähert sich die Tiefe des Kanals 64 der Achsfläche 63 asymptotisch an und läuft langsam aus, so dass ab einer festgelegten Winkelstellung, die sich aus der Länge des Kanals 64 ergibt, die Einmündung 92 durch die plan aufliegende Achsfläche 63 verschlossen wird. Der Flexionskanal 64 erstreckt sich nicht über den gesamten Umfang des Schwenkkolbens bzw. der Achsfläche 63, sondern nur über einen geringen Teil des Umfanges, so dass nur für einen Teilweg der Verschwenkung eine strömungstechnische Verbindung des Verbindungskanals 9 mit der entsprechenden Teilkammer bereitgestellt wird. Alternativ oder ergänzend zu einer geraden Ausgestaltung der Kanäle können diese auch mit einer Steigung versehen sein und sich wendelartig an dem Grundkörper bzw. in der Achsfläche 63 erstrecken, um eine gezielte Querschnittsverengung der Einmündungen 91, 92 zu erzielen.

In der Figur 4 ist ebenfalls der axial versetzt angeordnete Extensionskanal 65 zu erkennen, der eine nicht konstante Tiefe aufweist, so dass der freie Strömungsquerschnitt in Abhängigkeit von der Winkelstellung variabel ist. Auch hier läuft an dem Ende des Extensionskanals 65 dieser in die Achsfläche 63 aus, so dass die korrespondierende Einmündung 91 ab einer bestimmten Gelenkstellung durch die Achsfläche 63 verschlossen wird.

Eine Variante der Ausgestaltung des Schwenkkolbens 6 ist in der Figur 4a dargestellt, in der die Gesamte axiale Länge der Achsfläche 63 mit einer strömungsquerschnittsbestimmenden Kontur 64 ausgestattet ist, so dass man nicht mehr von einem Kanal im engeren Sinne sprechen kann. Auch in Extensionsrichtung kann eine entsprechend breite Kontur 65 ausgebildet sein, um schwenkwinkelabhängig und schwenkrichtungsabhängig variable Dämpfungen bereitzustellen. Wird nachfolgen von einem Kanal gesprochen, so ist allgemein eine strömungsquerschnittsbestimmende Kontur gemeint, deren Breite sich auch über die gesamte axiale Länge der Achsfläche 63 erstrecken kann.

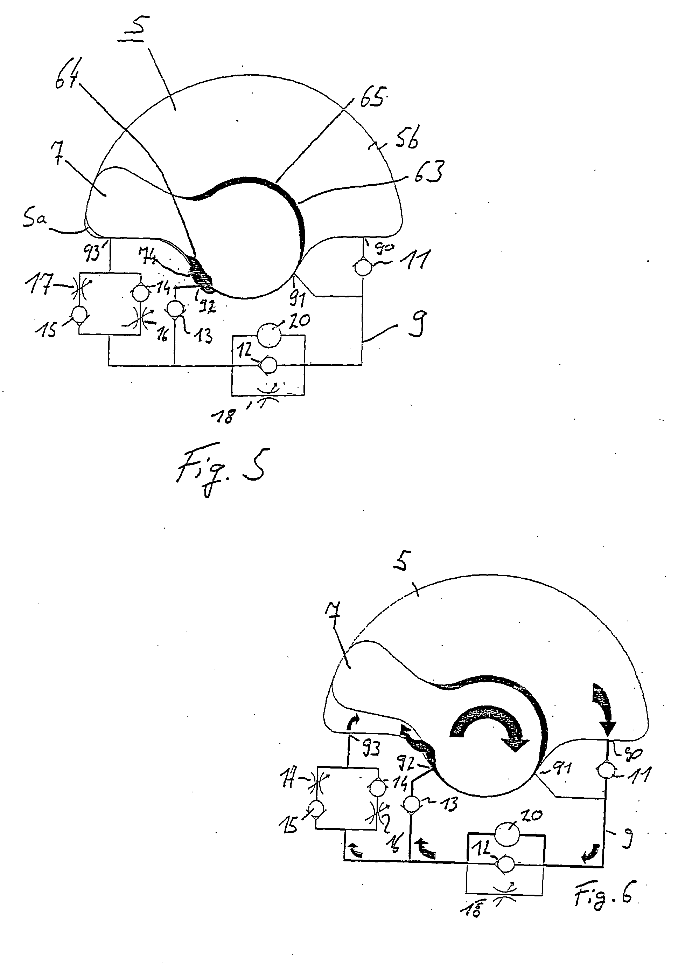

Die weitere hydraulische Schaltung und die Anordnung weiterer Ventile oder Drosseln ist in den Figuren 5 bis 10 dargestellt. Der Verbindungskanal 9 mit seinen Einmündungen 90, 91, 92, 93 in die Verdrängerkammer 5 verbindet die beiden Teilkammern 5a und 5b miteinander. In der Figur 5 ist zu erkennen, dass der Flexionskanal 64 kürzer als der Extensionskanal 65 ist und eine andere Kontur aufweist, so dass unterschiedliche Drosselkonturen bereitgestellt werden. Durch die Kontur der Kanäle 64, 65 ist es möglich, eine Grundform einer Dämpfungskurve der Rotationshydraulik innerhalb des Prothesengelenkes festzulegen, die durch weitere Maßnahmen eingestellt oder angepasst werden kann, beispielsweise indem das Dämpfungsgrundniveau angehoben oder abgesenkt wird. Ebenfalls ist in der Figur 5 zu erkennen, dass eine strömungsquerschnittsbestimmende Kontur 74 in der Verdrängerkammer 5 ausgebildet ist, die dem Flexionskanal 64 gegenüberliegt. Diese Kontur 74 kann als Kanal ausgebildet sein oder sich über die gesamte eaxial Länge der Verdrängerkammer 5 erstrecken. Ebenfalls ist es möglich, dass in der Verdrängerkammer 5, genauer dem der Achsfläche 63 gegenüberliegenden Bereich der Verdrängerkammer 5, ein Kanal oder eine Kontur dem Extensionskanal 65 oder der Extensionskontur gegenüberliegend ausgebildet ist, der oder die in der Figur 5 nicht dargestellt ist.

In dem dargestellten Ausführungsbeispiel gemäß der Figur 5 befindet sich das Prothesengelenk in einer Extensionsstellung. Die Kolbenplatte 7 minimiert das Volumen der Extensionskammer 5a, während das Volumen der Flexionskammer 5b maximal ist. Der Verbindungskanal 9 zwischen der Extensionskammer 5a und der Flexionskammer 5b als Teilkammern der Verdrängerkammer 5 zweigt sich auf, so dass zwei Einmündungen 90, 91 in die Flexionskammer 5b münden, während in die Extensionskammer 5a die Einmündungen 92, 93 münden. Von der Einmündung 90 führt der Verbindungskanal 9 über ein Rückschlagventil 11 zu einem Standphasenventil 20, das parallel zu einem Rückschlagventil 12 und einem Standphasendrosselventil 18 geschaltet ist. Nach dem ersten Rückschlagventil 11 mündet ein Zuführkanal von der Einmündung 91, die dem Extensionskanal 65 zugeordnet ist, in den Verbindungskanal 9.

Nach dem Standphasenventil 20 führt der Verbindungskanal 9 zu einem Rückschlagventil 13, das eine Extensionsbewegung sperrt. Hierzu parallel ist eine Ventilanordnung mit parallel geschalteten, gegenläufig orientierten Rückschlagventilen 14, 15 und jeweils seriell dazu geschalteten Flexions- und Extensionsventilen 17, 16 angeordnet. Die beiden Kanäle münden in die Einmündungen 92, 93.

Die Einmündungen 90, 91, 92, 94 müssen nicht unbedingt an den der Kobenplatte 7 gegenüberliegenden Wandungen der Verdrängerkammer 5 angeordnet sein, ebenfalls ist es möglich, dass die Einmündungen 90, 93 an der mantelförmigen, gebogenen Fläche, die dem distalen Ende der Kolbenplatte 7 gegenüberliegt, ausgebildet sein, so dass die Einmündung 90, 93 von dem Schwenkkolben 6 ab einer gewählten Stellung verschlossen oder über einen nicht dargestellten Kanal mit der Verdrängerkammer 5 verbunden wird.

In der Figur 6 ist der Beginn der Flexionsbewegung dargestellt. Die Kontur 74 in der Verdrängerkammer 5 ist in den Figuren 6 bis 10 nicht mehr dargestellt, ebenso ist eine korrespondierende Extensionskontur nicht dargestellt. Grundsätzlich gelten die nachfolgenden Ausführungen auch für eine Kombination der Kanäle 64, 65 bzw. Konturen 64, 65 mit zugeordneten Kanälen 74 oder Konturen in dem Verdrängerkammergehäuse. Ausgehend von der Extensionsstellung gemäß Figur 5 wird bei einer Flexion das Fluid, insbesondere das Hydraulikfluid, zunächst durch die stets offene Einmündung 90 durch das Rückschlagventil 11 in den Verbindungskanal 9 geleitet. Die zweite Einmündung 91, die dem Extensionskanal 65 zugeordnet ist, ist dabei noch durch die Achsfläche 63 verschlossen. Von dem Rückschlagventil 11 strömt das Fluid durch das Schaltventil 20, mit dem es möglich ist, eine maximale, hohe Dämpfung in Beugerichtung zu erzeugen, die unabhängig von den Konturen der Kanäle 64, 65, 74 und damit unabhängig von den freien Strömungsquerschnitte der Kanäle 64, 65, 74 über den gesamten Bewegungsbereich des Schwenkkolbens 6 und damit der Gelenkeinrichtung funktioniert. Nach dem Schaltventil 20 strömt das Fluid nahezu ungehindert durch das Rückschlagventil 13, die Einmündung 92 und den Flexionskanal 64 und die Kontur 74 in die Extensionskammer 5a. Ein weiterer Teilstrom strömt durch das Rückschlagventil 15 und ein einstellbares Flexionsventil 17 durch die zweite Einmündung 93 in die Extensionskammer 5a. Das Fluid läuft somit am Anfang der Flexion parallel durch zwei Kanäle, einmal über die Kontur durch den Kanal 64 und die Kontur 74 auf dem Schwenkkolben 6 und zusätzlich durch das Flexionsdrosselventil 17. Durch die zusätzliche Öffnung und Bereitstellung eines Strömungsquerschnittes in der Einmündung 92 wird eine sehr leichte Einleitung der Schwungphase erreicht, da das Fluid nahezu widerstandslos durch das Rückschlagventil 13 in die Extensionskammer 5 einströmen kann.

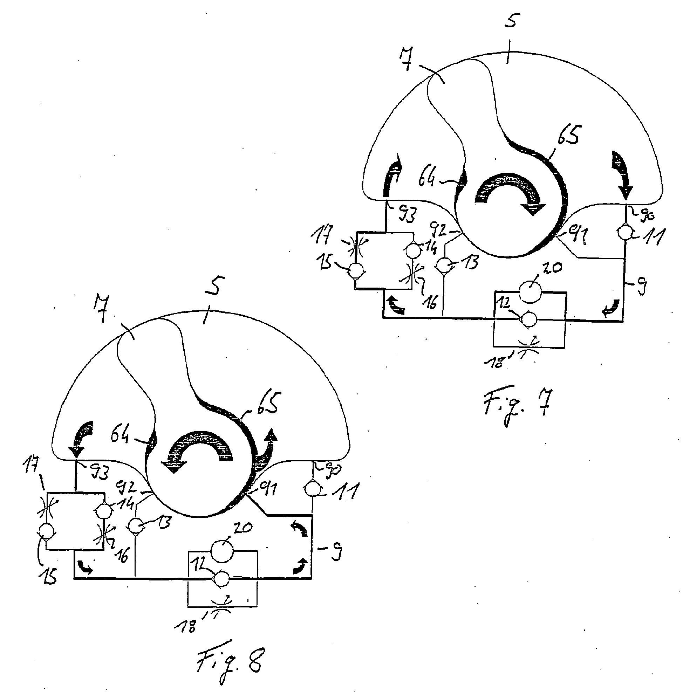

Bei einer weiteren Verschwenkung des Schwenkkolbens 6 verschließt die Achsfläche 63 die Einmündung 92, wie es in der Figur 7 dargestellt ist. Der Fluidstrom kann somit nur noch über das Standphasenventil 20 und das Rückschlagventil 15 sowie das eingestellte Flexionsventil 17 erfolgen. Aufgrund des verringerten Gesamtströmungsquerschnittes erhöht sich die Flexionsdämpfung im Vergleich zu dem Beginn der Verschwenkung, da ein erhöhter Strömungswiderstand bereitgestellt wird. In dem Zwischenbereich, zwischen dem Beginn der Flexion und dem Sperren der Einmündung 92, kann eine Variation der Dämpfung durch die Kontur des Flexionskanals 64 und oder die Kontur 74 bewirkt werden.

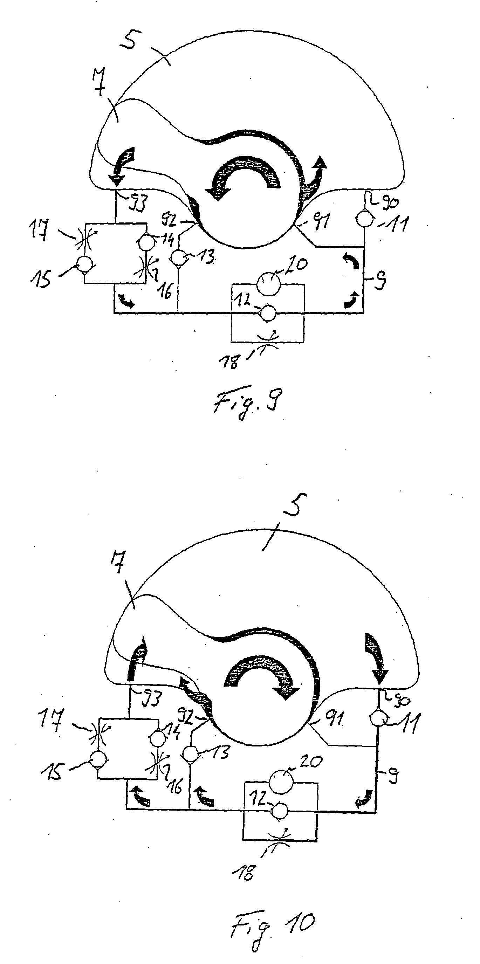

Die Dämpfung in der Extensionsbewegung ist in den Figuren 8 und 9 dargestellt. Die minimale Dämpfung in der Anfangsphase der Extension wird durch das Drosselventil 16 bestimmt. Durch das Drosselventil 16 fließt das Fluid durch das Rückschlagventil 12 über die Abzweigung des Verbindungskanals 9 durch die Einmündung 91 und den Extensionskanal 65 in die Flexionskammer 5b. Zusammen mit dem Drosselventil 16 und der Kontur des Extensionskanals 65 kann der Verlauf der Dämpfung eingestellt werden. Das Niveau der Dämpfung kann durch das einstellbare Drosselventil 16 ebenso wie durch die auf der Achsfläche 63 eingearbeitete Kontur des Kanals 65 bestimmt werden. Das Drosselventil 16 sowie der Kanal 65 sind seriell zueinander angeordnet.

Am Ende der Extension, wie sie in der Figur 9 angedeutet ist, verringert sich der Strömungsquerschnitt durch eine Abflachung des Extensionskanals 65 kontinuierlich, so dass sukzessiv die Mündung 91 verschlossen wird. Durch das langsame Verengen des freien Strömungsquerschnittes bis zu einem vollständigen Schließen wird eine zunehmende Dämpfung bereitgestellt, durch die es möglich wird, dass ein harter Endanschlag, auch bei unerwartet auftretenden hohen Kräften verhindert wird. Der Fluidstrom durch die sich verengende Kontur des Kanals 65 wird zum Ende der Extensionsbewegung deutlich reduziert, was durch eine dünnere Linie dargestellt ist.

In der Figur 10 ist eine Standphasenstellung des Prothesenkniegelenks gezeigt, bei der das Standphasenventil 20, das als Schaltventil ausgestaltet sein kann, geschlossen ist. Durch das Verschließen des Standphasenventils 20 entsteht für das Drosselventil 18 ein hoher Druck. Nach dem Standphasendrosselventil 18 verteilt sich das Fluid über die vorhandenen Verbindungen bis zu den Einmündungen 90, 91, jedoch ist der dadurch entstehende Widerstand deutlich geringer als an dem Standphasendrosselventil 18, so dass der Einfluss der Konturen der Kanäle 64, 65 verschwindend gering ist und vernachlässigt werden kann.

Sämtliche Drosselventile 16, 17, 18 können einstellbar ausgebildet sein, um eine Anpassung an den jeweiligen Prothesen- oder Orthesennutzer und die vorliegenden Einsatzbedingungen vornehmen zu können. Bevorzugt sind die Drosselventile 16, 17, 18 von außen einstellbar, so dass eine Anpassung leicht jeder Zeit erfolgen kann.

Der Fluidstrom und damit die Dämpfung des Prothesen- oder Orthesengelenkes wird während der Verschwenkbewegung, sowohl während der Flexion als auch während der Extension durch die in dem Schwenkkolben 6 angebrachte Kontur der Kanäle 64, 65 bestimmt oder zumindest mitbestimmt, ohne dass eine aufwendige motorische Verstellung oder eine bewegliche Lagerung gesonderter Stelleinrichtungen vorgesehen werden muss. Grundsätzlich ist es möglich und vorgesehen, dass die Konturen 64, 65, 74 oder Kanäle verstellbar ausgestaltet sind, beispielsweise über Drehhülsen oder Stellelemente, die die Kontur in axialer oder radialer Richtung ändern können. Trotz der Verstellbarkeit bleibt bei erfolgter Einstellung der relevante Verschwenkbereich oder Verschwenkwinkel, in dem der freie Strömungsquerschnitt beeinflusst wird, festgelegt.

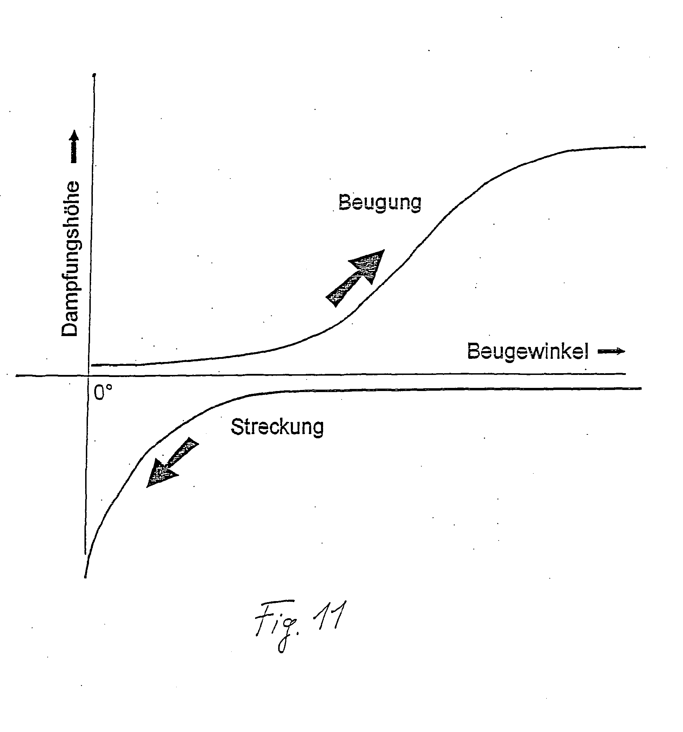

In der Figur 11 ist ein exemplarischer Verlauf der Dämpfungshöhe in Abhängigkeit vom Beugewinkel dargestellt. Die obere Kurve verdeutlicht den Verlauf der Dämpfung während der Flexion, also Beugung, während die untere Kurve den Dämpfungsverlauf während der Extension, also Streckung verdeutlicht. Der Kurvenverlauf korrespondiert zu der Ausgestaltung der Konturen 64, 65 in dem Schwenkkolben 6 gemäß den Figuren 6 bis 10. Während der Flexion wird mit einer zunächst relativ geringen Dämpfung das Beugen des Gelenkes, beispielsweise des Kniegelenkes, ermöglicht, indem das Fluid durch den Verbindungskanal 9 und die beiden Einmündungen 92, 93 im Wesentlichen ungehindert durchströmen kann. Ab einem bestimmten Beugewinkel wird die Einmündung 92 durch die Achsfläche 63 verschlossen, wodurch sich die Dämpfung über den Beugewinkel vergrößert. Die weitere Dämpfung erfolgt dann über das Flexionsdrosselventil 17, das einen Maximalwert festlegt.

Umgekehrt kann bei einer Streckung über nahezu den gesamten Bereich das Fluid durch die Einmündung 91 in die Teilkammer 5b einströmen, so dass ein geringer Widerstand aufgrund einer geringen Dämpfung vorhanden ist. Erst kurz vor Ende der Streckung, also in der Nähe Streckanschlages, verschließt die Achsfläche 63 die Einmündung 91 in zunehmendem Maße. Die Zunahme der Dämpfung wird durch eine kontinuierliche Verringerung des Strömungsquerschnittes durch die Kontur 65 im Endbereich bereitgestellt, bis diese Einmündung 91 komplett verschlossen wird. Nach einem vollständigen Verschließen der Einmündung 91 erfolgt ein Fluidstrom nur noch über Leckströme, die Grunddämpfung der Streckung wird über das Extensionsdrosselventil 14 in Verbindung mit der Kontur 65 eingestellt.

## Patentansprüche

1. Prothesen- oder Orthesengelenk mit einem ersten und einem zweiten Gelenkteil, die zueinander um eine Gelenkachse schwenkbar gelagert sind, und einem Schwenkkolben, der drehfest an einem der Gelenkteile angeordnet und in einer ein Fluid enthaltenden Verdrängerkammer geführt ist, wobei der Schwenkkolben die Verdrängerkammer in zwei Teilkammern unterteilt, die über einen Verbindungskanal zumindest über einen gewählten Verschwenkwinkelbereich des Schwenkkolbens miteinander verbunden sind, **dadurch gekennzeichnet, dass** zwischen dem Schwenkkolben (6) und einer Verdrängerkammerwandung zumindest eine strömungsquerschnittbestimmende Kontur (64, 65) ausgebildet ist, die über einen gewählten Verschwenkwinkel in strömungstechnischer Verbindung mit dem Verbindungskanal (9) und der Verdrängerkammer (5) steht und in Abhängigkeit von der Winkelstellung des Schwenkkolbens (6) einen unterschiedlichen freien Strömungsquerschnitt für den Durchtritt des Fluides bereitstellt.

2. Prothesen- oder Orthesengelenk nach Anspruch 1, **dadurch gekennzeichnet, dass** zwei Konturen (64, 65) in dem Schwenkkolben (6) eingearbeitet sind, die den Einmündungen (91, 92) des Verbindungskanals (9) zugeordnet sind.

3. Prothesen- oder Orthesengelenk nach Anspruch 1 oder 2, **dadurch gekennzeichnet, dass** die Konturen (64, 65) für die Extension und die Flexion unterschiedlich ausgebildet sind.

4. Prothesen- oder Orthesengelenk nach einem der voranstehenden Ansprüche, **dadurch gekennzeichnet, dass** die Kontur (64, 65) in einer Achsfläche (63) des Schwenkkolbens (6) und/oder in einer Verdrängerkammerwandung ausgebildet oder eingearbeitet ist.

5. Prothesen- oder Orthesengelenk nach einem der voranstehenden Ansprüche, **dadurch gekennzeichnet, dass** die Kontur (64, 65) in Abhängigkeit von der Winkelstellung des Schwenkkolbens (6) die Einmündung (91, 92) in einem unterschiedlichen Maß überdeckt.

6. Prothesen- oder Orthesengelenk nach einem der voranstehenden Ansprüche, **dadurch gekennzeichnet, dass** die Kontur (64, 65) die Einmündung (91, 92) überdeckend ausgebildet ist und der Konturgrund in Abhängigkeit von der Winkelstellung des Schwenkkolbens (6) einen unterschiedlichen Abstand zu der Einmündung (91, 92) aufweist.

7. Prothesen- oder Orthesengelenk nach einem der voranstehenden Ansprüche, **dadurch gekennzeichnet, dass** weitere Einmündungen (90, 93) des Verbindungskanals (9) in die Teilkammern (5a, 5b) vorhanden sind.

8. Prothesen- oder Orthesengelenk nach einem der voranstehenden Ansprüche, **dadurch gekennzeichnet, dass** in dem Verbindungskanal (9) zumindest ein Rückschlagventil (11, 12, 13, 14, 15) angeordnet ist.

9. Prothesen- oder Orthesengelenk nach einem der voranstehenden Ansprüche, **dadurch gekennzeichnet, dass** in dem Verbindungskanal (9) zumindest ein Schaltventil (20) angeordnet ist.

10. Prothesen- oder Orthesengelenk nach Anspruch 9, **dadurch gekennzeichnet, dass** das Schaltventil (20) unabhängig von der Kontur (64, 65) über den gesamten Bewegungsbereich des Schwenkkolbens (6) wirksam ist.

11. Prothesen- oder Orthesengelenk nach einem der voranstehenden Ansprüche, **dadurch gekennzeichnet, dass** in dem Verbindungskanal (9) zumindest eine einstellbare Drossel (16, 17, 18) angeordnet ist.

12. Prothesen- oder Orthesengelenk nach Anspruch 11, **dadurch gekennzeichnet, dass** die Drossel (6, 17, 18) von außen verstellbar ausgebildet ist.

13. Prothesen- oder Orthesengelenk nach einem der voranstehenden Ansprüche, **dadurch gekennzeichnet, dass** die Kontur (64, 65) als Kanal ausgebildet ist.
